# FASCICULE DE BREVET EUROPEEN

(11) **EP 1 513 482 B1**
(45) Date de publication et mention de la délivrance du brevet: **14.04.2010**
(21) Numéro de dépôt: 03756039.8
(22) Date de dépôt: 02.06.2003
(51) Int. Cl.: A61K 6/00, A61K 8/60, A61Q 5/08, A61Q 19/02, A61P 17/00

(54) **UTILISATION PAR VOIE TOPIQUE D'AU MOINS UN OLIGONUCLEOTIDE D'ARN DOUBLE BRIN (DS RNA) ANTI-TYROSINASE**
TOPISCHE ANWENDUNG MINDESTENS EINES DOPPELSTRÄNGIGEN RNA-OLIGONUCLEOTIDS (DS RNA) GEGEN TYROSINASE
TOPICAL USE OF AT LEAST ONE DOUBLE-STRANDED RNA OLIGONUCLEOTIDE (DS RNA) AGAINST TYROSINASE

(30) Priorité: 03.06.2002 FR 0206796; 10.06.2002 US 386720 P
(43) Date de publication de la demande: 16.03.2005
(73) Titulaire: L'Oréal, 75008 Paris (FR)
(72) Inventeur: DURANTON, Albert, F-78600 Maison Laffite (FR); COLLIN-DJANGONE, Christine, F-60110 Amblainville (FR); PRUCHE, Francis, F-60300 Senlis (FR); SIMONNET, Jean-Thierry, F-75011 Paris (FR)
(74) Mandataire: Bernstein, Claire Jacqueline
(86) Numéro de dépôt international: PCT/FR2003/001648
(87) Numéro de publication internationale: WO 2003/101376

(56) Documents cités:
- WO-A-01/36646
- WO-A-01/58918
- MIYAGISHI MAKOTO ET AL: "U6 promoter-driven siRNAs with four uridine 3' overhangs efficiently suppress targeted gene expression in mammalian cells." NATURE BIOTECHNOLOGY. UNITED STATES MAY 2002, vol. 19, no. 5, mai 2002 (2002-05), pages 497-500, XP002245728 ISSN: 1087-0156
- TUSCHL T: "RNA interference and small interfering RNAs" CHEMBIOCHEM - A EUROPEAN JOURNAL OF CHEMICAL BIOLOGY, WILEY VCH, WEINHEIM, DE, vol. 2, no. 4, avril 2001 (2001-04), pages 239-245, XP002961590 ISSN: 1439-4227
- FIRE A: "RNA-triggered gene silencing" TRENDS IN GENETICS, ELSEVIER SCIENCE PUBLISHERS B.V. AMSTERDAM, NL, vol. 15, no. 9, 1 septembre 1999 (1999-09-01), pages 358-363, XP004176656 ISSN: 0168-9525 cité dans la demande
- CHO KYUNG A ET AL: "Senescent phenotype can be reversed by reduction of caveolin status." JOURNAL OF BIOLOGICAL CHEMISTRY, vol. 278, no. 30, 25 juillet 2003 (2003-07-25), pages 27789-27795, XP002259038 ISSN: 0021-9258
- ASAI MASASHI ET AL: "Putative function of ADAM9, ADAM10, and ADAM17 as APP alpha-secretase." BIOCHEMICAL AND BIOPHYSICAL RESEARCH COMMUNICATIONS. UNITED STATES 31 JAN 2003, vol. 301, no. 1, 31 janvier 2003 (2003-01-31), pages 231-235, XP002245729 ISSN: 0006-291X
- ELBASHIR S.M. ET AL: 'Duplexes of 21-nucleotide RNAs mediate interference in cultured mammalian cells' NATURE vol. 411, 2001, LONDON, pages 494 - 498
- ELBASHIR S.M. ET AL: 'Analysis of gene function in somatic mammalian cells using small interfering RNAs' METHODS vol. 26, no. 2, Février 2002, SAN DIEGO, CALIF, pages 199 - 213, XP002251055

## Description

L'invention se rapporte à une nouvelle utilisation d'au moins un oligonucléotide d'ARN double brin (dsRNA) pour une application par voie topique ainsi qu'à des compositions topiques comprenant dans un milieu physiologiquement acceptable au moins un oligonucléotide d'ARN double brin encapsulé.

L'invention se rapporte également à un procédé de traitement cosmétique utilisant au moins un oligonucléotide d'ARN double brin.

La présente invention se rapporte à une nouvelle utilisation d'au moins un oligonucléotide d'ARN double brin (dsRNA) pour une application par voie topique ainsi qu'à des compositions à usage topique comprenant dans un milieu physiologiquement acceptable au moins un oligonucléotide d'ARN double brin encapsulé.

De nombreuses substances sont utilisées par voie topique pour palier ou prévenir certaines altérations esthétiques ou certains désordres de la peau ou de ses annexes. Parmi elles, les substances présentant une réelle spécificité biologique sont plus particulièrement recherchées.
En effet, plus les substances présenteront une activité spécifique, telle qu'une activité biologique spécifique, moins elles risqueront de provoquer des effets secondaires indésirables. Par exemple, dans une composition visant à éclaircir le teint ou à prévenir la pigmentation indésirable, on préférera utiliser une substance dont l'action sera ciblée sur une enzyme comme la tyrosinase. On peut également citer à titre d'exemple l'utilisation d'une substance agissant de façon spécifique sur certaines métalloprotéinases ou certaines élastases pour la préparation de composition pour prévenir le vieillissement.

Jusqu'à présent, ce problème de spécificité était résolu grâce à l'utilisation d'oligonucléotides d'ARN ou d'ADN natif ou ADN anti-sens. Une telle utilisation comporte cependant des limitations.

En effet, l'utilisation d'oligonucléotides (tels que, par exemple, ceux décrits dans la demande de brevet WO 01/58918), d'ADN, d'ARN non spécifique ou d'ADN anti-sens dans des formulations topiques n'a pas permis d'obtenir une efficacité souhaitable. En particulier, à la surface de la peau, des RNases dégradent l'ARN natif, les quantités atteignant les ARNm cibles ne peuvent être suffisantes pour obtenir l'effet recherché.
Une autre limitation de l'utilisation des oligonucléotides simple brin tels que ceux mentionnés ci-dessus est le risque que se produisent d'éventuels repliements secondaires (ou réappariements secondaires) de l'oligonucléotide. De ce fait, l'administration *in vivo* d'oligonucléotides simple brin (tels que des oligonucléotides d'ADN anti-sens) est souvent inefficace. De plus, la stabilité des effets *in vivo* des oligonucléotides simple brin est courte à cause d'une dégradation intracellulaire rapide des oligonucléotides, et d'une demi-vie extracellulaire courte *in vivo* (Khan A & al J Drug Target 2000 8 319-334).

Il existe ainsi un besoin de composition résolvant à la fois les difficultés d'administration ciblée des actifs ainsi que le problème de spécificité et adapté à une application topique.

La connaissance des oligonucléotides d'ARN double brin, aussi appelés dsRNA pour « double-stranded » RNA, est établie depuis longtemps chez les plantes et les organismes eucaryotes (Haines D.S. et al., J. Cell Biochem. 1991 ; 46 :9-20), ils ont pour rôle d'inhiber l'expression d'un gène spécifique (Fire A., Trends in Genetic 1999 ; 15 : 358-363).

L'utilisation de dsRNA à des fins thérapeutiques a été décrite dans la demande de brevet WO01/36646 qui propose une méthode d'inhibition de gènes impliqués dans des maladies, en particulier des cancers. Les exemples de mise en oeuvre de cette méthode proposent l'inhibition de l'expression d'un gène dans des cellules indifférenciées, telles que des cellules embryonnaires, ou des ovocytes, par l'injection intracellulaire de dsRNA visant le gène à inhiber. L'objectif est d'obtenir un effet systémique. Les modes d'administration envisagés concernent des administrations par voie générale.

Contrairement aux compositions pharmaceutiques proposées dans ce document, une formulation topique doit atteindre une cellule différenciée spécifique, tout en n'agissant qu'à un niveau superficiel, c'est à dire au niveau de la peau ou ses annexes (derme, épiderme et phanères), voire même uniquement à la surface de la peau en ciblant les microorganismes eucaryotes qui la recouvrent. On peut également vouloir rechercher un effet au niveau d'un type cellulaire cutané spécifique, tel que le fibroblaste, le mélanocyte, le kératinocyte, la cellule de Langherans, la cellule endothéliale, impliqué dans des altérations esthétiques ou des désordres dermatologiques particuliers.
Dans le cadre de la présente invention, on a maintenant trouvé qu'il est possible d'utiliser des oligonucléotides d'ARN double brin pour inhiber de façon spécifique l'expression de certaines protéines de cellules différenciées, en particulier de cellules de la peau, et donc pour la préparation de compositions à usage topique externe.
C'est pourquoi la présente invention a pour objet l'utilisation pour une application par voie topique d'au moins un oligonucléotide d'ARN double brin qui est active sur des cellules différenciées telles que les cellules du derme et de l'épiderme (mélanocytes, fibroblastes, kératinocytes...).

L'utilisation selon l'invention présente à la fois les propriétés mentionnées ci-dessus et permet de surmonter les difficultés rencontrées jusqu'à ce jour :
- elle vise spécifiquement l'expression de protéines des cellules du derme et de l'épiderme ou de micro-organismes eucaryotes à la surface de la peau, le ciblage des cellules pourra, en particulier, se faire par le choix d'un enrobage permettant le relargage des oligonucléotides d'ARN double brin dans la cellule visée ;
- Les oligonucléotides d'ARN double brin permettent l'inhibition spécifique d'un gène (ou de plusieurs dans le cas d'une composition associant des oligonucléotides d'ARN double brin ayant des séquences différentes et visant des ARNm codant pour des protéines différentes) ;
- les oligonucléotides d'ARN double brin, grâce à leur structure en duplex, sont résistants aux RNases (enzymes de dégradation de l'ARN), ils ne sont pas dégradés à la surface de la peau ;
- en raison de leur structure en duplex, ces oligonucléotides d'ARN double brin ne peuvent pas subir de repliement et s'autoapparier ;
ainsi, la bio-disponibilité du produit actif, c'est-à-dire la quantité d'oligonucléotide d'ARN double brin produisant l'effet recherché qui parvient aux cellules, est nettement améliorée et permet l'utilisation de quantité d'oligonucléotide d'ARN double brin plus faible.

Les compositions contenant l'ARN double brin selon l'invention seront particulièrement adaptées à un usage cosmétique ou dermatologique, puisqu'elles permettent d'atteindre la cible cellulaire sans mode d'administration invasif, et sont actives sans avoir à pénétrer dans le noyau cellulaire.
C'est par le biais d'un mécanisme complexe au niveau cytoplasmique qu'est mise en jeu la machinerie cellulaire induisant une inhibition de l'expression de l'ARNm correspondant.
L'administration dans une cellule d'oligonucléotide d'ARN double brin, induit un phénomène d'extinction post-transcriptionnel de l'ARNm qu'il vise.

Le mécanisme moléculaire mis en jeu fait intervenir des séquences de 21 à 23 nucléotides comprises dans les oligonucléotides d'ARN double brin, ces séquences étant responsables de la spécificité de la séquence de l'ARNm. Ces oligonucléotides d'ARN double brin, sont aussi appelés dsRNA ou encore siRNA (pour «short interfering» RNA, voir Tuschl T. Chem. Biochem. 2001 ;2 :239-245). Le mécanisme fait intervenir un complexe dsRNA-protéine formé de manière ATP dépendante et qui déclenche une réaction de dégradation spécifique de l'ARNm visé (Nykanen A & al Cell 2001 107 309-321).

L'objet de la présente invention se rapporte tout d'abord à une utilisation pour une application par voie topique d'au moins un oligonucléotide d'ARN double brin comportant généralement au moins 10 nucléotides, notamment entre 12 et 40 nucléotides, préférentiellement de 20 à 25 nucléotides; on préférera tout particulièrement les oligonucléotides d'ARN double brin comportant entre 21 et 23 nucléotides.

Par oligonucléotide d'ARN double brin, on entend une séquence d'acide ribonucléique ayant une structure de double hélice avec une séquence substantiellement identique à au moins une partie de l'ARN messager visé.

La séquence des oligonucléotides d'ARN double brin selon l'invention dérive généralement d'une séquence endogène, c'est-à-dire qu'elle représente tout ou partie de séquences nucléotidiques mammifères ou de micro-organismes eucaryotes. Il peut s'agir de séquence de gène, de séquence d'ADN codant (ADNc) produits par exemple avec la reverse transcriptase à partir d'ARN messager (ARNm) issu d'un mammifère, il peut également s'agir de séquence de gène de levure.
En particulier, lorsque la séquence de l'oligonucléotide d'ARN double brin correspond à tout ou partie de la séquence d'un gène, on préférera utiliser les séquences d'un ou plusieurs exons du gène visé.

Les oligonucléotides d'ARN double brin selon l'invention pourront être des oligonucléotides d'ARN double brin comportant un ou plusieurs nucléotides modifiés par substitution, délétion ou insertion, ces modifications seront telles que la séquence de l'oligonucléotide d'ARN double brin lui permettra de reconnaître spécifiquement un fragment de l'ARNm cible du mécanisme de dégradation.

Les oligonucléotides d'ARN double brin pourront également présenter un squelette modifié lui conférant, par exemple, une meilleure stabilité.
Par exemple, les liaisons phosphodiester des brins d'ARN naturels peuvent être modifiées pour inclure au moins un atome d'azote ou de soufre. De plus, les oligonucléotides d'ARN double brin selon l'invention peuvent comprendre des bases autres que les 4 bases classiques.
La structure en double brin de l'oligonucléotide d'ARN double brin peut être obtenue par l'appariement de deux brins simples d'ARN complémentaires ou bien l'oligonucléotide d'ARN double brin peut être obtenu par le repliement et l'appariement d'un unique brin simple d'ARN "autocomplémentaire", c'est-à-dire comprenant deux fragments de séquences complémentaires pouvant s'apparier par repliement du brin simple pour former une double hélice.

Par oligonucléotide d'ARN double brin substantiellement identique à un fragment de gène, on entend un oligonucléotide d'ARN double brin dont la séquence présente un degré d'homologie (pourcentage de bases d'acide nucléiques identiques entre deux séquences, voir les méthodes de calcul proposées par Atschul et al. J. Molec. Biol. 1990, 215:403) avec le fragment dudit gène compris entre 80 et 100% et préférentiellement d'au moins 90%.

Dans un mode de réalisation préféré de l'invention, l'oligonucléotide d'ARN double brin présente des extrémités non appariées de 2 à 6 nucléotides.

L'oligonucléotide d'ARN double brin selon l'invention peut également être modifié par l'addition d'un polyéthylène glycol tel que défini par Garrett et al. (Bioorg. Med. Chem. 2000 July 8(7): 19779-97) afin d'améliorer l'efficacité de sa transfection dans la cellule hôte.

Les oligonucléotides d'ARN double brin selon l'invention peuvent être synthétisés selon de nombreuses méthodes de synthèse *in vivo* ou *in vitro* de façon manuelle ou automatique.

Les méthodes de synthèse *in vitro* peuvent être chimiques ou enzymatiques, par exemple en utilisant une ARN polymérase (à titre d'exemple de T3, T7 ou SP6) qui réalisera la transcription d'un modèle de séquence d'ADN (ou d'ADNc) choisi.

De nombreuses méthodes de synthèse *in vivo* d'ARN double brin sont décrites dans la littérature, elles peuvent être réalisées dans divers types cellulaires de bactéries ou d'organismes supérieurs (Sambrook et al. Molecular Cloning, A Laboratory Manual, Second Edition (1989), DNA cloning, volume I and II, D. N. Glover (ed. 1985), Oliginucleotide Synthesis, M. J. Gaits (ed. 1984), Nucleic Acid Hybridation, B. D. Hames and S. J. Higgins (ed.1984), Transcription and Translation B. D. Hames and S. J. Higgins (ed.1984), Animal Cell Culture, R. I. Freshney (ed. 1986), Immobilised Cells and Enzymes, IRL Press (1986), B. Pertal, A Practical Guide to Molecular Cloning (1984), Gene Transfer Vectors for Mammalian Cells, J. H. Miller and M. P. Calos, Cold Spring Harbor Laboratory (ed. 1987), Methods in Enzymology, vol.154, Wu and Grossman, and 155, Wu, Mayer and Walker (1987), Immunochemical Methods in Cell and Molecular Biology, Academic Press, London, Scopes (1987), Protein Purification: Principle and Practice, 2nd ed., Springer-Verlag, N.-Y. and Handbook of Experimental Immunology, vol.I-IV, C. D. Weir and C. C. Blackwell (1986)). On pourra également se référer aux méthodes de synthèses décrites dans les demandes de brevet WO01/36646 et WO01/75164.

La séquence des oligonucléotides d'ARN double brin utilisés sera choisie pour une utilisation topique spécifique, avec toute méthode de biologie moléculaire. Les ARNm des cibles biologiques d'intérêt dans le domaine cosmétique ou dermatologique peuvent être, à titre d'exemple, les protéines décrites ci-après, mais aussi tout ARNm codant pour d'autres protéines cutanées.

On pourra également utiliser une association de plusieurs oligonucléotides d'ARN double brin de séquence différente et ayant chacun une activité différente afin, par exemple, d'obtenir un effet complémentaire ou synergique.

En particulier, la présente invention se rapporte à l'utilisation par voie topique d'au moins un oligonucléotide d'ARN double brin capable d'inhiber l'expression d'un ARN messager codant pour une protéine exprimée par une cellule de la peau ou de ses annexes.

La présente invention se rapporte également à une composition cosmétique dans laquelle l'oligonucléotide d'ARN double brin est capable d'inhiber l'expression d'un ARN messager codant pour une protéine exprimée par un micro-organisme eucaryote se trouvant à la surface de la peau ou du cuir chevelu.

En particulier, la protéine de la peau est une protéine exprimée par des mélanocytes et/ou des kératinocytes et/ou des fibroblastes et/ou les cellules endothéliales et/ou les cellules immunitaires résidentes, telles que les cellules de Langherans. De façon préférée, il s'agira d'une protéine exprimée par les kératinocytes.
Les protéines et/ou les oligo-peptides, dont on peut réduire ou inhiber la synthèse, sont celles impliquées dans les phénomènes de prolifération ou de différenciation cellulaire, comme par exemple :
- les facteurs de croissance EGF, TNF-α, TGF, endothéline, NGF, HGF, IGF, VEGF ;
- les cytokines par exemple de type IL1, IL6, IL8 ... ;
- les récepteurs de type EGFr, TGFr, PAR, PPAR, FXR, RXR, CB1R, CB2R, VR1, CRAB2... ;
- les calcium binding proteins de type calmoduline, CLP, CLSP, celles de la famille des S100 proteins telles que S100A8, S100A9, S100A7
- la calcineurine ;
- les transglutaminases par exemple les transglutaminases 1, 3 ou 5 ;
- les protéines assurant la cohésion/jonction intercellulaire telles occludines, laminines, caveolines, desmogleines, desmocollines, corneodesmosines, plakoglobines, desmoplakines... ;
- les enzymes impliquées dans les modifications postraductionnelles des protéines telles que les phosphatases ou protein-phosphatases par exemple la calcineurine, les phosphorylases, les proteins kinases (ex : PKC), les glucosyl transferases, les peptidyl-arginine-deiminase ... ;
- les protéases (MMP par exemple 1, 2, 3, 9, elastases, protéases à acide aspartique comme cathepsine-E, cathepsine-D, proteases à cystéine de type cathepsin-L, B ou H, cathepsine L2, SCCL, chymotrypsine équivalentes par exemple de type SCCE (Kallicrein 7), trypsin-like par exemple de type SCTE (Kallicrein 5), urokinase, SASPase, Caspase plus particulièrement caspase 14, calpaïnes, les protéases impliquées dans l'hydrolyse de la filaggrine de type subtilisin-like proprotein convertase comme la furin, PACE4, PC5/6 et PC7/8, les protéases de la famille des proteases à serine de type transmembranaire par exemple la matriptase et/ou leurs inhibiteurs endogènes comme TIMP, PAI1, PAI2, antileukoprotease, Elafin, LEKTI, cystatin A, cystatin M/E... ;
- les exo et les endoglycosidases par exemple de type heparanase, hyaluronidases, chondroîtinases, aspartyl glucosaminidase, B glycosidase, a glycosidases ... et leurs inhibiteurs endogènes ;
- les enzymes du métabolisme des lipides telles que HMGCoA reductase, cholestérol sulfatases ou sulfo transferases, sphingomyélinases, ceramidases... ;
- la tyrosinase, TRP-1 ou -2 ;
- les enzymes du métabolisme des eicosanoides comme, par exemple, les cyclooxygénases, lipoxygénases, les phospholipases, 15-PGDH... ;
- les enzymes du métabolisme hormonal telle la 5 α-réductase de type 1 ou 2 ;
- les protéines matricielles de type , élastines, collagène ... ;
- les protéines de différenciation kératinocytaire de type cytokératine ;
- les protéines impliquées dans l'hydratation de la peau telle que la filaggrine, les aquaporines... ;
- les protéines impliquées dans les défenses antibactériennes de la peau hBD2, hBD3, dermcidin, RNase 7 ... ;
D'autres exemples non limitatifs, de protéines ou de peptides dont on vise à inhiber l'expression et/ou l'activité sont donnés dans « Textbook of dermatology » ed RH Champion JL Burton, DA Burns, SM Breathnach sixth edition 1998 Blackwell Science LtD ISBN 0-632-03796-2.

Au niveau du derme, l'ARN messager, dont on peut réduire ou inhiber l'expression, peut être celui des fibroblastes ou d'autres cellules présentes dans le derme, les cellules des vaisseaux, les cellules des annexes épidermiques (exemples : sébocytes, glandes sudoripares,...), les cellules pouvant migrer dans le derme comme les cellules impliquées dans l'immunité ou l'inflammation.

Les protéines et/ou les oligo-peptides, dont on peut réduire ou inhiber la synthèse, sont celles impliquées dans les phénomènes de prolifération ou de différenciation cellulaire comme, par exemple, celles décrites pour les kératinocytes et plus encore :
- les facteurs de croissance EGF, TGF, endothéline, NGF, HGF, FGF...
- les phosphatases
- les transglutaminases
- les phosphorylases
- les protéines impliquées dans le renouvellement de la matrice extra cellulaire (protéases : métalloprotéases MMP, sérines protéases comme urokinase, tPA, les hyaluronidases
- les protéines de structure du derme comme le collagène ou de la substance fondamentale comme les protéines des protéoglycannes
- les protéines impliquées dans la maturation du derme comme la lysyl oxydase, la lysyl hydroxylase,

Au niveau de structures complexes comme le follicule pileux, on conçoit d'utiliser un ensemble de siRNA chacun dirigé sur un messager connu pour coder pour une protéine régulatrice et/ou de structure de la tige pilaire afin d'obtenir l'effet désiré :
- anti-pousse : par exemple, les protéines impliquées dans le cycle cellulaire et ou IGFrécepteur et/ou récepteur thyroïdien à T4 ;
- anti-chute : par exemple les siRNA dirigés contres des cytokines IL1, IL6, TNFalpha, MCP1 et/ou des protéases MMP urokinase et/ou lipoxygenase ;
- repousse : par exemple les protéines impliquées dans la dégradation de PGF2alpha et/ou 5alpha réductase ; les protéines impliquées dans la morphogénèse comme alpha3betal-integrine, beta-catenine, Laminine-10, LEF-1 ;
- mise en forme de la tige pilaire, comme le frisage ou le défrisage : par exemple les protéines impliquées dans la différenciation de la tige pilaire telles que les hair-kératines acides ou basiques ainsi que les enzymes associées à la réticulation des protéines de la tige (ex : thiol-oxydoréductases et transglutaminase 3 et 5) ;.
On peut se référer comme liste non limitative de ce type de protéines à « Jamora C, DasGupta R, Kocieniewski P, Fuchs E. Nature 2003 Mar 20;422(6929):317-22 »..).

Au niveau de structures complexes comme la glande sébacée, on conçoit d'utiliser un ensemble de siRNA chacun dirigé contre les protéines impliquées dans la synthèse du sébum comme par exemple HMG Co a réductase, squalène synthase.

Pour tous ces types cellulaires, on peut envisager, selon l'invention, d'utiliser des siRNA dirigés contre l'expression de protéines et/ou des oligo-peptides pathologiques, en particulier celles correspondant à l'action de virus (ex. HPV de verrues) ou à des cellules cancéreuses ou celles sur-exprimées dans certaines pathologies. On peut citer par exemple certaines cytokines (par exemple IL1, TNFalpha -308, TNFbeta +252), des protéines réceptrices (par exemple Toll-like recepteurs TLRs), certaines protéines impliquées dans la prolifération comme la phosphatidylinositol 3-kinase, les molécules d'adhésion (par exemple CDw60), des protéases, notamment les sérines protéases (stratum corneum chimotrypsine enzyme SCCE) ou les métalloprotéases (en particulier MMP-9, MMP-19 impliquées dans le psoriasis), ou encore des protéines liées au calcium (calmodulin like serine protease CLSP).
Pour tous ces types cellulaires, on peut également envisager d'utiliser selon l'invention des siRNA dirigés contre l'expression de protéines et/ou des oligo-peptides impliqués dans la désactivation d'un médicament destiné au traitement de la peau, par exemple des protéines de détoxification comme le le cytochrome P450 ou le cytochrome CYP2S1 dans le cas du psoriasis.

Pour tous ces types cellulaires, on peut envisager, selon l'invention, d'utiliser des siRNA dirigés contre l'expression de protéines et/ou des oligo-peptides induits par des altérations externes, par exemple des siRNA dirigés contre P2X purinergique récepteur après altération mécanique / chimique de la barrière cornée.

Les micro-organismes se trouvant à sa surface, dont on peut réduire ou inhiber la synthèse de protéines et/ou d'oligo-peptides, sont des Eucaryotes comme, par exemple, les levures, les champignons. On peut citer de manière non limitative :
- les organismes dermatophytes, qui sont les agents responsables des mycoses (les espèces Trichophyton dont T. rubrum, T. mentagrophytes ). Les dermatophytes sont des champignons filamenteux kératinophiles, c'est à dire ayant un tropisme préférentiel pour les phanères (poils et ongles) et la couche cornée.
   Trois types de dermatophytes sont à l'origine des dermatophytoses (ou dermatophyties).
   1. Les dermatophytes anthropophiles : ceux-ci sont strictement d'origine humaine. Les agents sont par exemple :
      Trichophyton rubrum, Trichophyton interdigitale, Trichophyton Violaceum, T. Rosaceeum, T. Tonsurans, T. Soudanensae, Trichophyton Schoenleinii, Epidermophyton Floccosum Microsporum Audouinii
   2. Les dermatophytes zoophiles qui sont transmis à l'homme par les animaux. Les agents responsables sont par exemple Microsporum canis, Trichophyton Mentagrophytes Trichophyton Ochraceum.
   3. Les dermatophytes géophiles qui sont transmis à l'homme par le sol. Le principal agent est le microsporum gypseum.
- Les non dermatophytes (les espèces Candida comme C. albicans ...les espéces Scopulariopsis comme S. brevicaulis, Malassezia spp). Ce sont les levures représentées par le genre candida et par Malassezia furfur (anciennement appelé pityrosporon). Le candida affectionne la peau, les phanères et les muqueuses. Malassezia furfur, saprophyte fréquent de la peau surtout séborrhéique est l'agent du pityriasis versicolor.
   S'il est tout à fait normal que des particules de peau morte se détachent du cuir chevelu comme du reste de la peau, il arrive que ce processus permanent de renouvellement de l'épiderme prenne des proportions gênantes. Inesthétiques, les pellicules sont aussi parfois accompagnées de problèmes plus sérieux du cuir chevelu tels qu'irritations, rougeurs et démangeaisons. On parle alors de dermatite séborrhéique, une affection cutanée favorisée par le champignon Pityrosporum ovale.
- Les moisissures, plus rarement impliquées dans les affections de la couche cornée. Elles sont responsables de certaines onychomycoses et des mycoses invasives.

On conçoit selon l'invention une inhibition de protéines spécifiques de ces organismes comme par exemple la squalène epoxidase, la cytochrome P450-dependente 14-alpha-sterol demethylase de C. albicans, des protéines de détoxification des agents anti-mycotiques comme les esterases de Trichophyton rubrum, les aspartique protéinases, la Phospho-lipase D, la Phospholipase B de Candida, les 2,3-oxidosqualene cyclases comme 22,23-epoxy-2-aza-2,3-dihydrosqualene (EAS) and azasqualene alcohol (ASA). On trouvera une liste plus complète à titre d'exemples non limitatif, d'organismes eucaryotes, dans « Textbook of dermatology » ed RH Champion JL Burton, DA Burns, SM Breathnach sixth edition 1998 Blackwell Science LtD ISBN 0-632-03796-2

Plus généralement selon l'invention les siRNA sont choisis et/ou associés selon l'effet escompté par voie topique. On conçoit aisément avec l'état de l'art d'utiliser une méthode moléculaire de criblage des ARN messagers induits et/ou réprimés par un pharmacophore ou par une cause d'effets indésirables (méthode des DNA array) pour déterminer le choix et l'assemblage des siRNA correspondant à l'effet à obtenir et/ou à empêcher.

Les dsRNA utilisés seront choisis pour une utilisation topique spécifique, avec toute méthode de biologie moléculaire convenant et tel que cela se fait pour tout ARN messager en particulier selon sa structure primaire. Les ARNm des cibles biologiques d'intérêt en cosmétique peuvent être, à titre d'exemple, et ceci dans une liste non limitative: tyrosinase, TRP-1, élastase, hyaluronidase, métalloprotéase, HmoCoA réductase, 5 α-réductase, SCCE, NO synthase, urokinase, ARNm de protéines épidermiques.....

Dans un exemple de réalisation de l'invention, on utilisera un oligonucléotide d'ARN double brin capable d'inhiber l'expression d'un ARN messager codant pour la tyrosinase.
En particulier, l'oligonucléotide d'ARN double brin est tel que son brin 5' sens a pour séquence SEQ ID n°1 : 5'-UGCACCACUUGGGCCUCAAdTdT et que son brin 5' antisens a pour séquence SEQ ID n°2 : 5'-UUGAGGCCCAAGUGGUGCAdTdT.

L'utilisation selon l'invention peut également comprendre un oligonucléotide d'ARN double brin capable d'inhiber l'expression d'un ARN messager codant pour la TRP-1 (tyrosinase related protein 1).

De telles utilisations par voie topique sont adaptées à la dépigmentation et/ou au blanchiment de la peau et/ou des phanères.

Dans un autre exemple de réalisation de l'invention, on utilisera un oligonucléotide d'ARN double brin capable d'inhiber l'expression d'un ARN messager codant pour l'enzyme elastase neutrophile.
En particulier, l'oligonucléotide d'ARN double brin est tel que son brin 5' sens a pour séquence SEQ ID n°3 : 5'-CGGCUACGACCCCGUAAACdTdT et que son brin 5' antisens a pour séquence SEQ ID n°4 : 5'-GUUUACGGGGUCGUAGCCGdTdT.

Une telle utilisation par voie topique est destinée à prévenir et/ou lutter contre les signes du vieillissement cutané tels que la perte de fermeté et/ou de souplesse de la peau et/ou l'atrophie de la peau et/ou la formation des rides et ridules.

Dans un autre exemple de réalisation de l'invention, on utilisera un oligonucléotide d'ARN double brin capable d'inhiber l'expression d'un ARN messager codant pour l'hyaluronidase.

Une telle utilisation par voie topique est destinée à prévenir et/ou lutter contre les signes du vieillissement cutané tels que la perte de fermeté et/ou de souplesse de la peau et/ou l'atrophie de la peau et/ou la formation des rides et ridules.

Dans un autre exemple de réalisation de l'invention, on utilisera un oligonucléotide d'ARN double brin capable d'inhiber l'expression d'un ARN messager codant pour une métalloprotéinase.

Les métalloprotéinases (MMP) sont notamment décrites dans l'article de Y. HEROUY et al., European Journal of Dermatology, n° 3, vol. 10, Avril-Mai 2000, pp. 173-180.

La famille des métalloprotéinases est ainsi constituée de plusieurs groupes bien définis basés sur leurs ressemblances en terme de structure et de spécificité de substrat (voir Woessner J. F., Faseb Journal, vol. 5, 1991, 2145). Parmi ces groupes, on peut citer les collagénases destinées à dégrader les collagènes fibrillaires (MMP-1 ou collagénase interstitielle, MMP-8 ou collagénase de neutrophile, MMP-13 ou collagénase 3, MMP-18 ou collagénase 4), les gélatinases qui dégradent le collagène de type IV ou toute forme de collagène dénaturé (MMP-2 ou gélatinase A (72 kDa), MMP-9 ou gélatinase B (92 kDa)), les stromélysines (MMP-3 ou stromélysine 1, MMP-10 ou stromélysine 2, MMP-11 ou stromélysine 3) dont le large spectre d'activité s'adresse aux protéines de la matrice extracellulaire telles que les glycoprotéines (fibronectine, laminine), les protéoglycannes, etc., la matrilysine (MMP-7), la métalloélastase (MMP-12) ou encore les métalloprotéinases membranaires (MMP-14, MMP-15, MMP-16 et MMP-17).

Les métalloprotéinases (MMPs) sont les membres d'une famille d'enzymes protéolytiques (endoprotéases) qui possèdent un atome de zinc coordonné à 3 résidus cystéine et une méthionine dans leur site actif et qui dégradent les composants macromoléculaires de la matrice extracellulaire et des lames basales à pH neutre (collagène, élastine, etc ...). Très largement répandues dans le monde vivant, ces enzymes sont présentes, mais faiblement exprimées, dans des situations physiologiques normales comme la croissance des organes et le renouvellement des tissus. Leur surexpression chez l'homme et leur activation sont cependant liées à de nombreux processus qui impliquent la destruction et le remodelage de la matrice. Cela entraîne par exemple une résorption non contrôlée de la matrice extracellulaire.

Une telle utilisation par voie topique est destinée à prévenir et/ou lutter contre les signes du vieillissement cutané tels que la perte de fermeté et/ou de souplesse de la peau et/ou l'atrophie de la peau et/ou la formation des rides et ridules, elles sont également adaptées à une utilisation visant à induire et/ou stimuler la croissance des cheveux et/ou des poils et/ou freiner leur chute.

Dans un autre exemple de réalisation de l'invention, on utilisera un oligonucléotide d'ARN double brin capable d'inhiber l'expression d'un ARN messager codant pour la HMG-CoA reductase.
En particulier, l'oligonucléotide d'ARN double brin est tel que son brin 5' sens a pour séquence SEQ ID n°5 : 5'-AGCCGCGAGGGUCGUCCAAdTdT et que son brin 5' antisens a pour séquence SEQ ID n°6 : 5'-UUGGACGACCCUCGCGGCUdTdT.

Une telle utilisation par voie topique est destinée à prévenir et/ou lutter contre la production excessive de sébum ou de sueur au niveau des aisselles ou des pieds.

Dans un autre exemple de réalisation de l'invention, on utilisera un oligonucléotide d'ARN double brin capable d'inhiber l'expression d'un ARN messager codant pour la 5 α-reductase type I et/ou de type II, et préférentiellement la 5 α-reductase type I.

En particulier, l'oligonucléotide d'ARN double brin est tel que son brin 5' sens a pour séquence SEQ ID n°7 : 5'-CUGCAUCCUCCUGGCCAUGdTdT et que son brin 5' antisens a pour séquence SEQ ID n°8 : 5'-CAUGGCCAGGAGGAUGCAGdTdT.

Une telle utilisation par voie topique est destinée à traiter les désordres androgéno-dépendants, en particulier, à traiter l'hyperseborrhée et/ou l'acné et/ou induire et/ou stimuler la croissance des cheveux et/ou des poils et/ou freiner la chute des cheveux.

Dans un autre exemple de réalisation de l'invention, on utilisera un oligonucléotide d'ARN double brin capable d'inhiber l'expression d'un ARN messager codant pour la calmodulin-like skin protein (CLSP).
En particulier, l'oligonucléotide d'ARN double brin est tel que son brin 5' sens a pour séquence SEQ ID n°9: 5'-GGCUUUCUCCGCGGUUGACdTdT et que son brin 5' antisens a pour séquence SEQ ID n°10 : 5'-GUCAACCGCGGAGAAAGCCdTdT.

Une telle utilisation par voie topique est destinée à lutter contre les signes du vieillissement cutané et/ou lutter contre les effets néfastes des rayonnements ultraviolets et/ou traiter les peaux sèches.

Dans un autre exemple de réalisation de l'invention, on utilisera un oligonucléotide d'ARN double brin capable d'inhiber l'expression d'un ARN messager codant pour la NO synthase.

Une telle utilisation par voie topique est destinée à inhiber la différentiation et/ou la prolifération cellulaire et/ou inhiber la dégradation et/ou la destruction des cellules de la peau et ainsi lutter contre le vieillissement intrinsèque et/ou extrinsèque et/ou freiner la chute des cheveux. Ces compositions sont également adaptées au traitement des peaux sensibles et des érythèmes, en particulier photoinduits.

Dans un autre exemple de réalisation de l'invention, on utilisera un oligonucléotide d'ARN double brin capable d'inhiber l'expression d'un ARN messager codant pour une sérine protéase telle que l'urokinase. Les compositions de ce type seront particulièrement adaptées au traitement cosmétique et à l'amélioration de l'aspect des peaux sèches et/ou irritées.

Dans d'autres exemples de réalisation de l'invention, on pourra utiliser un oligonucléotide d'ARN double brin capable d'inhiber l'expression d'un ARN messager codant pour :
- IL1 pour ses applications dans le domaine des peaux sensibles,
- la 5α réductase pour ses applications dans le domaine des peaux grasses,
- une lipase bactérienne pour ses applications visant à lutter contre la formation de pellicules, ou encore
- Iox12 et/ou cox2 et/ou IL1 et/ou TGFβ1pour des applications visant à lutter conter la chute des cheveux.

Pour l'utilisation selon l'invention, l'oligonucléotide d'ARN double brin pourra être présent dans une composition à une concentration comprise entre 5. 10⁻⁷ et 5 %, de préférence entre 0,0001 et 1 % d'oligonucléotide(s) d'ARN double brin en poids par rapport au poids total de la composition.

Une telle composition peut se présenter sous toutes les formes galéniques normalement utilisées pour ce type d'application, notamment sous forme d'une solution aqueuse ou huileuse, d'une émulsion huile-dans-eau ou eau-dans-huile ou multiple, d'une émulsion siliconée, d'une microémulsion ou nanoémulsion, d'un gel aqueux ou huileux ou d'un produit anhydre liquide, pâteux ou solide.

Dans un autre de ses objets, la présente invention se rapporte à une composition destinée à être appliquée topiquement comprenant dans un milieu physiologiquement acceptable un d'oligonucléotide d'ARN double brin encapsulé.

Dans un mode de réalisation préféré selon l'invention, la composition est telle que l'oligonucléotide d'ARN double brin est encapsulé dans le coeur ou la paroi d'un enrobage tel que des microsphères, des nanosphères, des oléosomes, des niosomes ou des nanocapsules.

Les oligonucléotides d'ARN double brin à une concentration molaire efficace dans une solution aqueuse peuvent être complexés avec une polyéthyléne imine (PEI) d'un poids moléculaire 200 à 100000 g/M. La nature de la PEI et sa proportion de 10⁻⁴ à 1% sont fonction du type de solution aqueuse initiale contenant les oligonucléotides d'ARN double brin et de la formulation cosmétique envisagée. La complexation pourra être objectivée par l'opalescence de la solution initiale.

Une fois la complexation réalisée, ce complexe est encapsulé dans des particules à coeur aqueux, comme des niosomes, ou complexé à la surface de vésicules ayant une surface lamellaire contenant un polymère ou un tensio actif lipophile cationique comme par exemple les PEI hydrophobés.

Ainsi, l'oligonucléotide d'ARN double brin peut être soit :
- complexé par un poly-cation (par exemple une PEI hydrophile ou du chitosane) ;
   A titre d'exemple, on pourra choisir les polycations dérivés de poly(4-vinylpyrridine) (Modulation of interaction of polycations with negative unilamellar lipid vesicles de Yaroslavov et al dans Colloids and surfaces B:Biointerfaces 16(1999)29-43) ; les polymères et copolymères de Lysine (cités dans Turbidometric analysis of polyelectrolyte complexes formed between poly(L-lysine) and DNA de Ward et al dans Colloids and surfaces B:Biointerfaces 16(1999)253-260) ; Les copolymères de vinylpyrrolidine et de vinylimidazole quaternisés tels que les Luviquat de BASF (pm entre 1000 et 1000000) ; les copolymères à base de chlorure de diallyl diméthylammonium tels que les Merquats de BASF (pm entre 10000 et 10 000 000) ; les polyglucoseamines comme le chitosane et ses dérivés ; les polyamines tels que la polyethylène imine, hydrophobées ou non ; les dérives de Guars comme par exemple les Jaguar de Rhodia ; les dérivés de cellulose cationiques tels que les Celquat de National Starch ou les Quarisoft d'Amerchol ; les copolymères de vinylpirrolidone et de diméthylaminopropyl methacrylamide comme les Gafquat de ISP ; les dérivés cationiques de l'acide acrylique comme le HYPAN QT 100 de Kingston ; les Salcare SC92, SC95 et 96 de Allied Colloide, le Plex4739L de Rohm Gmbh ; les dérivés de diuréthane et polyuréthane tel que le Polyderme PPI-SA, le Foamox PPI-SA, le Foamtaine PPI-SA15 et le Foamquat PPI-SA de Alzo, les copolyamine commes le polyquart H-81 de Henkel ;
- ce complexe peut être alors encapsulé dans des particules à coeur aqueux comme les liposomes et en particulier les niosomes (voir la demande de brevet EP 0 582 503 ou le brevet US 5,439,672) ;
- complexé à la surface de vésicules ayant une surface lamellaire contenant un polymère ou un tensio-actif lipophile cationique.

Dans ce dernier cas, les tensio-actifs lipophiles cationiques peuvent être plus particulièrement choisis dans le groupe formé par les sels d'ammonium quaternaire, les amines grasses et leurs sels.

Les sels d'ammonium quaternaire sont par exemple : 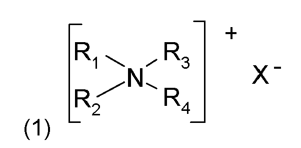 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl-ou-alkylarylsulfonates. Comme sels d'ammonium quaternaire de formule (II), on préfère, d'une part, les chlorures de tétraalkylammonium comme par exemple les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthyl-ammonium, de cétyltriméthyl-ammonium, de benzyl diméthyl stéaryl-ammonium ou encore, d'autre part, le chlorure de stéaramidopropyldiméthyl (myristyl acetate) ammonium vendu sous la dénomination «CERAPHYL 70» par la société VAN DYK.
(2) les sels d'ammonium quaternaire de l'imidazolinium, comme par exemple ceux de formule suivante : 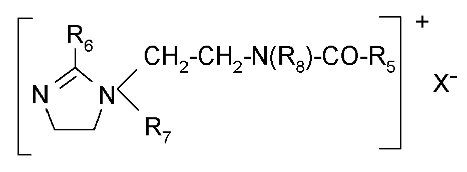 dans laquelle R₅ représente un radical alcényle ou alkyle comportant de 8 à 30 atomes de carbone, par exemple dérivé des acides gras du suif ; R₆ représente un atome d'hydrogène, un radical alkyle comportant de 1 à 4 atomes de carbone ou un radical alcényle ou alkyle comportant de 8 à 30 atomes de carbone ; R₇ représente un radical alkyle comportant de 1 à 4 atomes de carbone ; R₈ représente un atome d'hydrogène, un radical alkyle comportant de 1 à 4 atomes de carbone ; X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone, par exemple dérivés des acides gras du suif, R₇ désigne un radical méthyle, R₈ désigne l'hydrogène. Un tel produit est par exemple vendu sous la dénomination «REWOQUAT W 75» par la société REWO.
(3) les sels de diammonium quaternaire de formule : 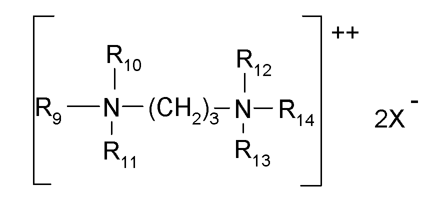 dans laquelle R₉ désigne un radical aliphatique comportant environ de 16 à 30 atomes de carbone ; R₁₀, R₁₁, R₁₂, R₁₃, et R₁₄ sont choisis parmi l'hydrogène ou un radical alkyle comportant de 1 à 4 atomes de carbone ; et X est un anion choisi dans le groupe des halogénures, acétates, phosphates, nitrates et méthylsulfates. De tels sels de diammonium quaternaire comprennent notamment le dichlorure de propanesuif diammonium.

L'oligonucléotide d'ARN double brin peut également être complexé à la surface de globules huileux cationiques, quelques soit leur taille (voir les demandes de brevet EP 1 010 413, EP 1 010 414, EP 1 010 415, EP 1 010 416, EP 1 013 338, EP 1 016 453, EP 1 018 363, EP 1 020 219, EP 1 025 898, EP 1 120 101, EP 1 120 102, EP 1 129 684, EP 1 160 005 et EP 172077).

L'oligonucléotide d'ARN double brin peut également être complexé à la surface de nanocapsules ou nanoparticules pourvues d'un enrobage lamellaire (Voir EP 0 447 318 et EP 0 557 489) et contenant un tensio-actif cationique à la surface (voir les références citées précédemment pour les tensio-actifs cationiques).

En particulier, les vésicules préférées selon la présente invention sont :
- les niosomes décrits dans le brevet EP 0 582 503 ;
- les sphérules de lipides dont la méthode de synthèse est décrite dans le brevet US 5,021,200 ;
- les niosomes huile dans eau décrits dans le brevet US 5,489,426 ;
- les nano-émulsions proposées dans le brevet EP 0 879 589.

On utilisera préférentiellement les lipides suivants : la PEI hydrophobée par greffage de chaînes alkyles de C10 à C22 (de 5 à 50 %) insaturées ou non, ramifiées ou non, la stéarylamine ou le Chlorure de Behenyl de triamonium.

On préférera plus particulièrement les vésicules ayant un diamètre inférieur ou égale à 2 µm et préférentiellement compris entre 50 nm et 1 µm.

On peut également utiliser des méthodes physiques d'administration intra-cutanée comme un système de propulsion gazeuse ou un patch à micro aiguilles.

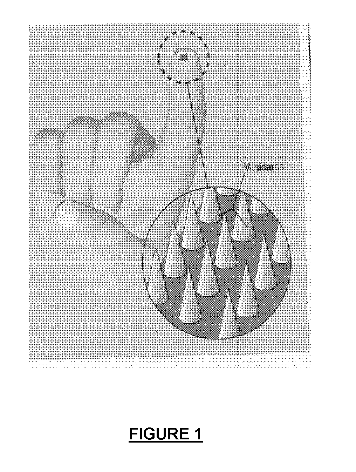

Sur la figure 1, qui représente un patch à microaiguilles, les minidards (cônes) ont une hauteur correspondant à la profondeur à laquelle doit arriver le dsRNA encapsulé dans une vésicule (épiderme superficiel, épiderme profond, derme superficiel...). La taille du patch peut, par exemple, être adaptée à la surface d'une tache pigmentaire ou d'une ride.

De façon plus détaillée, la composition destinée à être appliquée topiquement peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau.

Ainsi, la composition selon l'invention pourra en plus de l'oligonucléotide d'ARN double brin contenir au moins un actif choisi parmi : les α-hydroxyacides ; l'acide salicylique et ses dérivés tels que l'acide n-octanoyl-5-salicylique ; l'HEPES ; la procystéine ; l'O-octanoyl-6-D-maltose ; le sel disodique d'acide méthyl glycine diacétique ; les céramides ; les stéroïdes tels que la diosgénine et les dérivés de la DHEA ; l'acide kojique ; le N-éthyloxycarbonyl-4-para-aminophénol ; l'acide ascorbique et ses dérivés ; les extraits de myrtille ; les rétinoïdes et en particulier le rétinol et ses esters ; les polypeptides et leurs dérivés acylés ; les phytohormones ; les extraits de levure *Saccharomyces cerevisiae* ; les extraits d'algues ; les extraits de *Vitreoscilla filiformis ;* les extraits de soja, de lupin, de maïs et/ou de pois ; l'alvérine et ses sels, en particulier le citrate d'alvérine ; le resvératrol ; les caroténoïdes et en particulier le lycopène ; le tocophérol et ses esters ; le co-enzyme Q10 ou ubiquinone ; les xanthines et en particulier la caféine et les extraits naturels en contenant ; les extraits de petit houx et de marron d'Inde ; et leurs mélanges, sans que cette liste soit limitative.

La composition selon l'invention peut en outre contenir au moins un filtre UVA et/ou UVB. Les filtres solaires peuvent être choisis parmi les filtres organiques, les filtres inorganiques et leurs mélanges.

Comme exemples de filtres organiques actifs dans l'UV-A et/ou l'UV-B, on peut citer notamment, désignés ci-dessous par leur nom CTFA :
- les dérivés de l'acide para-aminobenzoïque : PABA, Ethyl PABA, Ethyl Dihydroxypropyl PABA, Ethylhexyl Diméthyl PABA vendu notamment sous le nom « ESCALOL 507 » par ISP, Glyceryl PABA, PEG-25 PABA vendu sous le nom « UVINUL P25 » par BASF,
- les dérivés salicyliques : Homosalate vendu sous le nom « EUSOLEX HMS » par RONA/EM INDUSTRIES, Ethylhexyl Salicylate vendu sous le nom « NEO HELIOPAN OS » par HAARMANN et REIMER, Dipropyleneglycol Salicylate vendu sous le nom « DIPSAL » par SCHER, TEA Salicylate, vendu sous le nom « NEO HELIOPAN TS » par HAARMANN et REIMER,
- les dérivés du dibenzoylméthane : Butyl Methoxydibenzoylmethane vendu notamment sous le nom commercial « PARSOL 1789 » par HOFFMANN LA ROCHE, Isopropyl Dibenzoylmethane,
- les dérivés cinnamiques : Ethylhexyl Methoxycinnamate vendu notamment sous le nom commercial «PARSOL MCX» par HOFFMANN LA ROCHE, Isopropyl Methoxy cinnamate, Isoamyl Methoxy cinnamate vendu sous le nom commercial « NEO HELIOPAN E 1000 » par HAARMANN et REIMER, Cinoxate, DEA Methoxycinnamate, Diisopropyl Methylcinnamate, Glyceryl Ethylhexanoate Dimethoxycinnamate,
- les dérivés de β,'-diphénylacrylate : Octocrylene vendu notamment sous le nom commercial « UVINUL N539 » par BASF, Etocrylene, vendu notamment sous le nom commercial « UVINUL N35 » par BASF,
- les dérivés de la benzophénone : Benzophenone-1 vendu sous le nom commercial « UVINUL 400 » par BASF, Benzophenone-2 vendu sous le nom commercial « UVINUL D50 » par BASF, Benzophenone-3 ou Oxybenzone, vendu sous le nom commercial « UVINUL M40 » par BASF, Benzophenone-4 vendu sous le nom commercial « UVINUL MS40 » par BASF, Benzophenone-5, Benzophenone-6 vendu sous le nom commercial « HELISORB 11 » par NORQUAY, Benzophenone-8 vendu sous le nom commercial « SPECTRA-SORB UV-24 » PAR AMERICAN CYANAMID, Benzophenone-9 vendu sous le nom commercial« UVINUL DS-49» par BASF, Benzophenone-12,
- les dérivés du benzylidène camphre : 3-Benzylidene camphor, 4-Methylbenzylidene camphor vendu sous le nom « EUSOLEX 6300 » par MERCK, Benzylidene Camphor Sulfonic Acid, Camphor Benzalkonium Methosulfate, Terephthalylidene Dicamphor Sulfonic Acid, Polyacrylamidomethyl Benzylidene Camphor,
- les dérivés du phényl benzimidazole : Phenylbenzimidazole Sulfonic Acid vendu notamment sous le nom commercial « EUSOLEX 232 » par MERCK, Benzimidazilate vendu sous le nom commercial commercial « NEO HELIOPAN AP » par HAARMANN et REIMER,
- les dérivés de la triazine : Anisotriazine vendu sous le nom commercial «TINOSORB S » par CIBA GEIGY, Ethylhexyl triazone vendu notamment sous le nom commercial «UVINUL T150 » par BASF, Diethylhexyl Butamido Triazone vendu sous le nom commercial « UVASORB HEB » par SIGMA 3V,
- les dérivés du phényl benzotriazole : Drometrizole Trisiloxane vendu sous le nom « SILATRIZOLE » par RHODIA CHIMIE ,
- les dérivés anthraniliques : Menthyl anthranilate vendu sous le nom commercial commercial « NEO HELIOPAN MA » par HAARMANN et REIMER,
- les dérivés d'imidazolines: Ethylhexyl Dimethoxybenzylidene Dioxoimidazoline Propionate,
- les dérivés du benzalmalonate : Polyorganosiloxane à fonctions benzalmalonate vendu sous la dénomination commerciale « PARSOL SLX » par HOFFMANN LA ROCHE,
- et leurs mélanges.

Les filtres UV organiques plus particulièrement préférés sont choisis parmi les composés suivants :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- Methylene bis-Benzotriazolyl Tetramethylbutylphenol,
- Drometrizole Trisiloxane,
- et leurs mélanges.

Les filtres inorganiques utilisables dans la composition selon l'invention sont en particulier les nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium. Des agents d'enrobage sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

De façon connue, la composition de l'invention peut contenir également les adjuvants habituels dans les domaines cosmétique et dermatologique, tels que les gélifiants hydrophiles ou lipophiles, les conservateurs, les antioxydants, les solvants, les tensioactifs, les épaississants, les parfums, les charges, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse ou dans la phase aqueuse. Ces adjuvants, ainsi que leurs concentrations, doivent être tels qu'ils ne nuisent pas aux propriétés avantageuses de l'acide pantéthéine sulfonique ou de ses sels.
Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans la composition de l'invention, on peut citer par exemple:
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone et la fraction liquide du beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

Comme émulsionnants et coémulsionnants utilisables dans l'invention, on peut citer par exemple les émulsionnants H/E tels que les esters d'acide gras et de polyéthylène glycol, notamment le stéarate de PEG-100, et les esters d'acide gras et de glycérine tels que le stéarate de glycéryle, ainsi que les émulsionnants E/H tels que le poly(méthylcétyl)(diméthyl)méthylsiloxane oxyéthyléné disponible sous la dénomination commerciale ABIL WE09 auprès de la société Degussa Goldschmidt ou le mélange de stéarate d'éthylène glycol acétyle et de tristéarate de glycéryle commercialisé par la société Guardian sous la dénomination commerciale UNITWIX.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme charges qui peuvent être utilisées dans la composition de l'invention, on peut citer par exemple, outre les pigments, la poudre de silice ; le talc ; l'amidon réticulé par l'anhydride octénylsuccinique commercialisé par la société National Starch sous la dénomination DRY FLO PLUS (28-1160) ; les particules de polyamide et notamment celles vendues sous la dénomination ORGASOL par la société Atochem ; les poudres de polyéthylène ; les micro-sphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition ou de la préparation selon l'invention.

L'homme du métier veillera à utiliser des ingrédients et des actifs qui ne nuiront pas à l'activité des oligonucléotides d'ARN double brin présents dans les compositions selon l'invention.

Un autre objet de l'invention se rapporte à un procédé de traitement cosmétique comprenant l'application topique sur une zone à traiter d'une composition comprenant dans un milieu physiologiquement acceptable au moins un oligonucléotide d'ARN double brin tel que défini précédemment. Avantageusement, la composition sera appliquée sur la peau et/ou le cuir chevelu et/ou les phanères, de façon unique ou répétée dans le temps.

Il pourra s'agir d'un procédé de dépigmentation et/ou de blanchiment de la peau ou des phanères caractérisé en ce qu'on applique sur la zone à traiter une composition selon l'invention, en particulier, une composition comprenant un oligonucléotide d'ARN double brin capable d'inhiber l'expression d'un ARN messager codant pour la tyrosinase et/ou un oligonucléotide d'ARN double brin capable d'inhiber l'expression d'un ARN messager codant pour la TRP-1.

L'invention se rapporte aussi à un procédé de traitement cosmétique pour prévenir et/ou lutter contre les signes cutanés du vieillissement caractérisé en ce qu'on applique sur la zone à traiter une composition comprenant un oligonucléotide d'ARN double brin capable d'inhiber l'expression d'un ARN messager codant pour la neutrophile élastase et/ou un oligonucléotide d'ARN double brin capable d'inhiber l'expression d'un ARN messager codant pour l'hyaluronidase et/ou un oligonucléotide d'ARN double brin capable d'inhiber l'expression d'un ARN messager codant pour une metalloprotéinase et/ou un oligonucléotide d'ARN double brin capable d'inhiber l'expression d'un ARN messager codant pour la HMG-CoA réductase et/ou un oligonucléotide d'ARN double brin capable d'inhiber l'expression d'un ARN messager codant pour la calmodulin-like skin protein (CLSP) et/ou un oligonucléotide d'ARN double brin capable d'inhiber l'expression d'un ARN messager codant pour la NO-synthase.

L'invention se rapporte également à un procédé de traitement cosmétique pour induire et/ou stimuler la croissance des cheveux et/ou des poils et/ou freiner la chute des cheveux caractérisé en ce qu'on applique sur la zone à traiter une composition comprenant un oligonucléotide d'ARN double brin capable d'inhiber l'expression d'un ARN messager codant pour une metalloprotéinase et/ou un oligonucléotide d'ARN double brin capable d'inhiber l'expression d'un ARN messager codant pour la 5 α-reductase type I ou II et/ou un oligonucléotide d'ARN double brin capable d'inhiber l'expression d'un ARN messager codant pour la NO-synthase.

Les exemples qui suivent sont destinés à illustrer l'invention. Dans ces exemples, on se référera aux figures suivantes:
- Figure 1: représente un patch à microaiguilles pouvant être utilisé pour l'administration d'oligonucléotides d'ARN double brin.
- Figure 2: extinction spécifique de l'expression de la tyrosinase dans les mélanocytes humains (MeWo) par ARN interférence

### Exemple 1 - inhibition de l'expression de la tyrosinase par ARN interférence

### Matériels et méthodes :

Les conditions expérimentales sont celles décrites par Tuschl et al. (Journal of Cell Science 2001, 114, 4557-4565).

Les mélanocytes (cellules MeWo) sont ensemencés 24 h avant transfection à 40 000 cellules/puits dans une plaque 24 puits dans 500 µl de milieu DMEM + 10% sérum. Par puits, 0.84 µg ou 2.52 µg de duplex siRNA (voir description ci-dessous), soit respectivement 60 pmoles de siRNA-Tyrosinase dans 3 µl d'annealing buffer (Dharmacon) ou 180 pmoles de siRNA-Tyrosinase dans 9 µl d'annealing buffer, sont utilisés. 3µl ou 9 µl de duplex siRNA-Tyrosinase à 20 µM sont dilués dans 50 µl d'Opti-MEM (Gibco). Dans un autre tube, 3 µl d'Oligofectamine (Invitrogen) sont dilués dans 12 µl d'Opti-MEM et incubés 10 minutes à température ambiante. Les 2 solutions sont mélangées et incubées 20 minutes à température ambiante avant d'être déposées sur les cellules. Les cellules sont alors incubées 96 h à 37°C avec un changement de milieu après 24 h puis lysées dans un tampon contenant 10 mM Tris-HCl pH7.2, 2% SDS, 1% Triton X-100, 10% glycérol + 1% protéase inhibiteur. 34 µg de protéines sont déposés par puits sur gel de polyacrylamide (NuPAGE Novex bis-Tris gels, *Invitrogen*)*,* séparés par électrophorèse et transferrés par Western Blot selon les instructions du fournisseur. La présence de tyrosinase sur la membrane est analysée par hybridation avec un anticorps anti-tyrosinase dilué au 1/500 (NCL-TYR, *Novocastra*) et la quantité protéique déposée est contrôlée par hybridation avec un anticorps anti-vimentine dilué au 1/40 000 (clone VIM3B4, *Cymbus Biotechnologies*). La membrane est rincée dans du PBS-Tween 20 et hybridée avec un anticorps secondaire anti-souris couplé à la peroxidase (Goat anti-mouse-HRP, Dako) dilué au 1/2000. La détection est réalisée à l'aide du kit « ECL Plus western blotting détection system » suivant les instructions du fournisseur (Amersham).

Les duplexes siRNA suivants sont sélectionnés à partir de la séquence codant pour l'ADNc de la tyrosinase humaine (Numéro d'accession M27160). Le numéro adjacent correspond à la position sur la séquence nucléique du 1^{er} nucléotide. Ils sont synthétisés par Dharmacon ou à partir d'oligonucléotides d'ADN fournis par Proligo et transcrits en ARN à l'aide du kit « siRNA construction Kit» (Ambion) suivant les instructions du fournisseur. Les 7 duplexes comprenant un brin sens ARN et un brin antisens ARN sont constitués par les séquences suivantes où AUG sont des bases ribonucléiques et dT une base déoxyribonucléique :
- siRNA-TYR266 duplex :
   5' brin sens siRNA (SEQ ID N°1) : UGCACCACUUGGGCCUCAAdTdT
   5' brin antisens siRNA (SEQ ID N°2) : UUGAGGCCCAAGUGGUGCAdTdT
- siRNA-TYR359 duplex :
   5' brin sens siRNA (SEQ ID N°11) : AAGUGUUUGAUGCUGGAGGdTdT
   5' brin antisens siRNA (SEQ ID N°12) : CCUCCAGCAUCAAACACUUdTdT
- siRNA-TYR690 duplex :
   5' brin sens siRNA (SEQ ID N°13) : : GCACCAGCUUUUCUGCCUUdTdT
   5' brin antisens siRNA (SEQ ID N°14) : AAGGCAGAAAAGCUGGUGCdTdT
- siRNA-TYR832 duplex :
   5' brin sens siRNA (SEQ ID N°15) : ACUGCACAGAGAGACGACUdTdT
   5' brin antisens siRNA (SEQ ID N°16) : AGUCGUCUCUCUGUGCAGUdTdT
- siRNA-TYR1110 duplex :
   5' brin sens siRNA (SEQ ID N°17) : GCACCAGCUUUUCUGCCUUdTdT
   5' brin antisens siRNA (SEQ ID N°18) : AAGGCAGAAAAGCUGGUGCdTdT
- siRNA-TYR1578 duplex :
   5' brin sens siRNA (SEQ ID N°19) : AGCAGCAUGCACAAUGCCUdTdT
   5' brin antisens siRNA (SEQ ID N°20) : AGGCAUUGUGCAUGCUGCUdTdT
- siRNA-TYR2120 duplex :
   5' brin sens siRNA (SEQ ID N°21) : AGCCUGACCUCACUCUAACdTdT
   5' brin antisens siRNA (SEQ ID N°22) : GUUAGAGUGAGGUCAGGCUdTdT

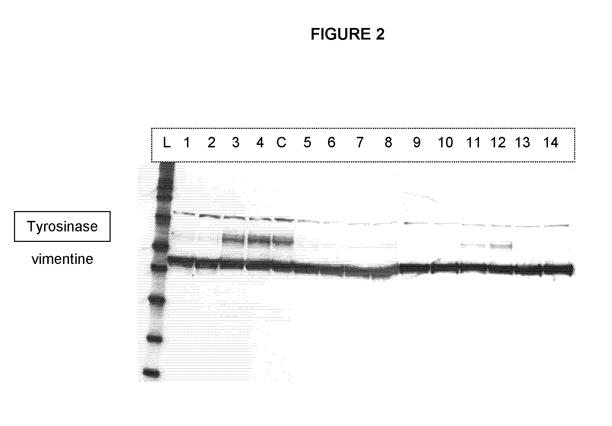

Les résultats sont représentés sur la figure 2.

Les pistes correspondent aux produits suivants
L : échelle de poids moléculaire
C : MeWo contrôle
1 : MeWo + siRNATYR266 0.84 µg
2 : MeWo + siRNATYR266 2.52 µg
3 : MeWo + siRNATYR359 0.84 µg
4 : MeWo + siRNATYR359 2.52 µg
5 : MeWo + siRNATYR690 0.84 µg
6 : MeWo + siRNATYR690 2.52 µg
7 : MeWo + siRNATYR832 0.84 µg
8 : MeWo + siRNATYR832 2.52 µg
9 : MeWo + siRNATYR1110 0.84 µg
10: MeWo + siRNATYR1110 2.52 µg
11 : MeWo + siRNATYR1578 0.84 µg
12 : MeWo + siRNATYR1578 2.52 µg
13 : MeWo + siRNATYR2120 0.84 µg
14 : MeWo + siRNATYR2120 2.52 µg

L'extinction de l'expression de la tyrosinase est vérifiée par Western blot. Il apparaît que, sur les 7 duplexes sélectionnés, 1 duplex est inefficace (siRNA 359), 1 duplex est faiblement efficace (siRNA 1578) et les 5 autres duplexes sont fonctionnels avec apparemment une très forte efficacité pour les duplexes 1110 et 2120 comme le montre l'absence de détection de la tyrosinase. On inhibe ainsi par « l'ARN interférence » l'expression de protéines par des cellules épidermiques, par des inhibiteurs spécifiques de la tyrosinase.

### Exemple 2 - Compositions

### Composition 1 : L'oligonucléotide d'ARN double brin est complexé à la surface de vésicules cationiques :

### Préparation des vésicules :

| | |
|---|---|
| Isostéarate de PEG 400 | 8 % |
| Polyéthylène Imine (PEI de PM 200 à 100000) amidifié par acide isostéarique | 2 % |
| Eau distillée | qsp100% |

Oligonucléotides d'ARN double brin de SEQ ID n°1 et SEQ ID n°2 avec un ratio dsRNA/PEI compris entre 0,1 et 1000.

### Etapes de réalisation des vésicules :

- solubilisation des lipides dans un mélange méthanol/chloroforme;
- évaporation du solvant à l'évaporateur rotatif sous pression réduite pour obtenir un film lipidique ;
- hydratation du film sous agitation Ultra Son (US) ;
- contrôle du diamètre des vésicules (moins de 1 µm, de préférence, de 200 nm) ;
- ajout de la solution de oligonucléotides d'ARN double brin.

### Composition cosmétique :

Les vésicules sont ensuite incorporées dans une formulation cosmétique adaptée à une application topique.

Une application quotidienne de préférence le soir de cette préparation permet l'atténuation des taches pigmentaires, une application régulière et durable conduit à la disparition de ces taches pigmentaires.

### Composition 2 : Le dsRNA est complexé à l'intérieur de vésicules cationiques

### A) Encapsulation d'un complexe polycation / oligonucléotides d'ARN double brin

| | |
|---|---|
| Stéarate de POE (8) | 5,5% |
| Cholestérol | 4% |
| Polyéthylène Imine (PM 200 à 100000) amidifié par acide isostéarique | 0.5% |

Solution aqueuse A : eau distillée 30%, PEI (PM 200 à 100000) 1% et oligonucléotide d'ARN double brin QSP.
Solution aqueuse B : eau distillée qsp 100%

### B) étapes de préparation des vésicules :

voir la préparation de la composition 1. Dans ce cas, l'étape d'hydratation se fait à la température adaptée au mélange lipidique et avec la solution A sous agitation ultrason puis s'ajoute une étape de dilution avec la solution aqueuse B.

### Composition cosmétique :

Les vésicules sont ensuite incorporées dans une formulation cosmétique adaptée à une application topique.

Ainsi, les compositions 1 et 2 peuvent ensuite être introduites de manière classique pour l'homme du métier dans la formulation suivante. Les ingrédients sont identifiés selon la nomenclature CTFA.

| | | |
|---|---|---|
| Glycérine | | 5 % |
| Conservateurs | | 1 % |
| Disodium EDTA | | 0,2 % |
| Carbomer | | 0,5 % |
| Triéthanolamine | | 0,25% |
| Ammonium polyacryloyldimethyl taurate* | | 0,3 % |
| Sodium hyaluronate | | 0,1 % |
| Huiles végétales | | 3 % |
| Acrylamide/sodium acryloyldimethyltaurate et isododécane et polysorbate-80** | | 2 % |
| Cyclopentasiloxane et dimethiconol | | 3 % |
| Suspension de vésicules selon exemples 1 ou 2 | | 1 à 25% |
| Eau | qsp | 100 % |

| | | |
|---|---|---|
| * Hostacerin AMPS fourni par CLARIANT ** Simulgel 600 fourni par SEPPIC | | |

### LISTE DE SEQUENCES

SEQ ID n°1 : 5'-UGCACCACUUGGGCCUCAAdTdT
SEQ ID n°2 : 5'-UUGAGGCCCAAGUGGUGCAdTdT
SEQ ID n°3 : 5'-CGGCUACGACCCCGUAAACdTdT
SEQ ID n°4 : 5'-GUUUACGGGGUCGUAGCCGdTdT
SEQ ID n°5 : 5'-AGCCGCGAGGGUCGUCCAAdTdT
SEQ ID n°6 : 5'-UUGGACGACCCUCGCGGCUdTdT
SEQ ID n°7 : 5'-CUGCAUCCUCCUGGCCAUGdTdT
SEQ ID n°8 : 5'-CAUGGCCAGGAGGAUGCAGdTdT
SEQ ID n°9 : 5'-GGCUUUCUCCGCGGUUGACdT
SEQ ID n°10 : 5'-GUCAACCGCGGAGAAAGCCdTdT.
SEQ ID n°11: 5'-AAGUGUUUGAUGCUGGAGGdTdT
SEQ ID n°12: 5'-CCUCCAGCAUCAAACACUUdTdT
SEQ ID n°13: : 5'-GCACCAGCUUUUCUGCCUUdTdT
SEQ ID n°14: 5'-AAGGCAGAAAAGCUGGUGCdTdT
SEQ ID n°15: 5'-ACUGCACAGAGAGACGACUdTdT
SEQ ID n°16: 5'-AGUCGUCUCUCUGUGCAGUdTdT
SEQ ID n°17: 5'-GCACCAGCUUUUCUGCCUUdTdT
SEQ ID n°18: 5'-AAGGCAGAAAAGCUGGUGCdTdT
SEQ ID n°19: 5'-AGCAGCAUGCACAAUGCCUdTdT
SEQ ID n°20: 5'-AGGCAUUGUGCAUGCUGCUdTdT
SEQ ID n°21: 5'-AGCCUGACCUCACUCUAACdTdT
SEQ ID N°22 : 5'-GUUAGAGUGAGGUCAGGCUdTdT

## Revendications

1. Utilisation d'au moins un oligonucléotide d'ARN double brin de séquence choisie parmi SEQ ID NO: 1, SEQ ID NO: 2, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, ou SEQ ID NO: 22, pour une application par voie topique pour inhiber l'expression d'une protéine de la peau ou de ses annexes, ladite protéine étant la tyrosinase.

2. Utilisation selon la revendication précédente, **caractérisée en ce que** l'oligonucléotide est un brin simple d'ARN autocomplémentaire apparié par repliement.

3. Composition topique comprenant dans un milieu physiologiquement acceptable au moins un oligonucléotide d'ARN double brin de séquence choisie parmi SEQ ID NO : 1, SEQ ID NO: 2, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, ou SEQ ID NO: 22, inhibant l'expression de la tyrosinase, encapsulé dans un enrobage.

4. Composition selon la revendication 4, **caractérisée en ce que** l'enrobage est choisi parmi les microsphères, les nanosphères, les oléosomes ou les nanocapsules.

5. Composition selon la revendication 4 ou 5, **caractérisée en ce que** l'enrobage a un diamètre inférieur ou égale à 2 µm.

6. Composition selon l'une quelconque des revendications 4 à 6, **caractérisée en ce que** le ou les oligonucléotide(s) d'ARN double brin représentent entre 5.10⁻⁷ et 5 % en poids par rapport au poids total de la composition.

7. Procédé cosmétique de dépigmentation et/ou de blanchiment de la peau ou des phanères, **caractérisée en ce que** l'oligonucléotide d'ARN double brin de séquence choisie parmi SEQ ID NO: 1, SEQ ID NO: 2, SEQ ID NO: 13, SEQ ID NO: 14, SEQ ID NO: 15, SEQ ID NO: 16, 20, SEQ ID NO : 21 ou SEQ ID NO: 22, est capable d"inhiber l'expression d'un ARN messager codant pour la tyrosinase.

## Claims

1. Nontherapeutic use of at least one double-stranded RNA oligonucleotide which has a sequence selected from SEQ ID NO: 1, SEQ ID NO: 2, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 22, for a topical application for depigmentating and/or bleaching the skin or the integuments via the inhibition of the expression of a protein of the skin or of its appendages, said protein being tyrosinase.

2. Use according to the preceding claim, **characterised in that** the oilgonucleotide is a self-complementary RNA single strand paired by folding.

3. Topical composition comprising, in a physiologically acceptable medium, at least one double-stranded RNA oligonucleotide which has a sequence selected from SEQ ID NO: 1, SEQ ID NO: 2, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 22, which inhibits the expression of tyrosinase, and which is encapsulated in a covering.

4. Composition according to Claim 3, **characterized in that** the covering is selected from microsphere, nanospheres, oleosomes or nanocapsules.

5. Composition according to Claim 3 or 4, **characterised in that** the diameter of the covering is less than or equal to 2 µm.

6. Composition according to any one of Claims 3 to 5, oligonucleotide(s) represent(s) between 5 × 10⁻⁷ and 5% by weight based on the total weight of the composition.

7. Cosmetic method for depigmenting and/or bleaching the skin or the integuments, **characterized in that** the double-stranded RNA oligonucleotide which has a sequence selected from SEQ ID NO: 1, SEQ ID NO: 2, SEQ ID NO: 13, SEQ ID NO: 14, SEQ ID NO: 15, SEQ ID NO: 16, 20, SEQ ID NO : 21 or SEQ ID NO: 22 is capable of inhibiting the expression of a messenger RNA encoding tyrosinase.

## Patentansprüche

1. Nichttherapeutische Verwendung mindestens eines doppelsträngigen RNA-Oligonucleotids einer Sequenz, die unter SEQ ID NO: 1, SEQ ID NO: 2, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 oder SEQ ID NO: 22 ausgewählt ist, für eine topische Anwendung zur Depigmentierung und/oder zum Bleichen der Haut oder der Hautanhangsgebilde durch Inhibierung der Expression eines Proteins der Haut oder der Hautanhangsgebilde, wobei es sich bei dem Protein um die Tyrosinase handelt.

2. Verwendung nach dem vorhergehenden Anspruch, **dadurch gekennzeichnet**, das das Oligonucleotid ein durch Faltung gepaarter, autokomplementärer RNA-Einfachstrang ist,

3. Topische Zusammensetzung, die in einem physiologisch akzeptablen Medium mindestens ein doppelsträngiges RNA-Oligonucleotid einer Sequenz enthält, die unter SEQ ID NO: 1, SEQ ID NO: 2, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 oder SEQ ID NO: 22 ausgewählt ist, das die Expression der Tyrosinase hemmt und in einer Umhüllung eingekapselt ist.

4. Zusammensetzung nach Anspruch 3, **dadurch gekennzeichnet, dass** die Umhüllung unter den Mikrosphären, Nanosphären, Oleosomen und Nanokapseln ausgewählt ist.

5. Zusammensetzung nach Anspruch 3 oder 4, **dadurch gekennzeichnet, dass** die Umhüllung einen Durchmesser kleiner oder gleich 2 µm aufweist.

6. Zusammensetzung nach einem, dar Ansprüche 3 bis 5, **dadurch gekennzeichnet, dass** das oder die RNA-Oligonucleotid(e) 5-10⁻⁷ bis 5 Gew.-%, bezogen auf das Gesamtgewicht der Zusammensetzung, ausmachen.

7. Kosmetisches Verfahren zur Depigmentierung und/oder zum Bleichen der Haut oder der Hautanhangsgebilde, **dadurch gekennzeichnet, dass** das doppelsträngige RNA-Oligonucleotid einer Sequenz, die unter SEQ ID NO: 1, SEQ ID NO: 2, SEQ ID NO: 13, SEQ ID NO: 1.4, SEQ ID NO: 15, SEQ ID NO: 16, SEQ ID NO: 17, SEQ ID NO: 18, SEQ ID NO: 19, SEQ ID NO: 20, SEQ ID NO: 21 oder SEQ ID NO: 22 ausgewählt ist, befähigt ist, die Expression einer Boten-RNA zu inhibieren, die die Tyrosinase kodiert.
